# EUROPEAN PATENT SPECIFICATION

(11) **EP 1 152 660 B1**
(45) Date of publication and mention of the grant of the patent: **29.01.2003**
(21) Application number: 00926510.9
(22) Date of filing: 04.05.2000
(51) Int. Cl.: A01N 25/34, A01N 25/18

(54) **MAT FOR DISPENSING VOLATILE MATERIALS**
MATTE ZUR ABGABE VON FLÜCHTIGEN STOFFEN
FILTRE POUR LA REPARTITION DE MATIERES VOLATILES

(30) Priority: 07.05.1999 US 307150
(43) Date of publication of application: 14.11.2001
(73) Proprietor: S. C. Johnson & Son, Inc., Racine, WI 53403 (US)
(72) Inventor: FLASHINSKI, Stanley, J., Racine, WI 53402 (US); VNUK, Nancy, J., South Milwaukee, WI 53172 (US); BOOTZ, Lori, J., West Allis, WI 53227 (US)
(74) Representative: Jones, Alan John
(86) International application number: PCT/US00/12107
(87) International publication number: WO 00/067574

(56) References cited:
- GB-A- 2 166 653
- PATENT ABSTRACTS OF JAPAN vol. 012, no. 372 (C-533), 5 October 1988 (1988-10-05) & JP 63 122603 A (OIKE IND CO LTD), 26 May 1988 (1988-05-26)
- DATABASE WPI Section Ch, Week 198238 Derwent Publications Ltd., London, GB; Class A32, AN 1982-80239E XP002147906 & JP 57 131550 A (NITTO ELECTRIC IND CO), 14 August 1982 (1982-08-14)

## Description

### BACKGROUND OF THE INVENTION

This invention relates to a method for dispensing volatile materials such as pest control materials, including but not limited to insecticides and insect repellents, and fragrances. More particularly, it relates to a method for dispensing volatile vapors from volatile material-containing mats that are employed in conjunction with electrical, gas, flammable liquid, or wax-fueled heaters or other sources of heat. One type of electrical heater used for this purpose is sold by S.C. Johnson & Son, Inc. under the trademark FUYI VAPE.

It is known in the art to impregnate a solid, porous cellulosic mat with a volatile material (US 4,974,797) or to place a volatile material in a pan-like metal structure (product sold by Bayer). These mats and pans are placed on heaters to cause the volatile material to vaporize into the atmosphere.

A problem with the metal pan-like structures is that for typical heaters they can cause a volatile material to be exposed to too much heat. This can cause the volatile to be used up too fast or be deteriorated or destroyed through thermal degradation.

The mats have similar problems and also have problems with respect to their being exposed to differing temperatures across a heater surface. Low-cost existing heaters often have hotter regions at certain points along their burner surface. The mats therefore can have uneven and inefficient vaporization.

The above problems are of increased concern for extended longevity products intended to be used for a week or more. Merely adding additional volatile to increase product capacity and longevity does not work well because prolonged exposure of volatiles to too high temperatures can degrade or destroy the volatile and because, with hot temperatures, a disproportionate amount of the volatile can be driven off initially, with an insufficient amount surviving to be released in useful amounts at a later time.

Another design consideration is that existing heaters, for safety and other reasons, often only accept slab-like inserts having a small cross-sectional shape, necessary to fit into a small heater loading port or opening. Thus, any solution to the extended longevity problem preferably takes into account size restrictions imposed by existing heaters.

Yet another critical design consideration is cost. Mats of this type are often used in countries that have very modest average annual incomes. To have much practical application in those countries, the mats must be inexpensive.

GB 2,166,653 (D1) discloses a mat impregnated with deodorant, disinfectant or pesticide and intended for use in electrically heated devices.

D1 discloses a method for dispensing volatile vapors comprising the steps of:
(a) providing a mat comprising a carrier layer having a permeable, porous, inert material imbibed with volatile material having a metal layer secured to the upper side of the carrier layer and/or a heat insulating coating secured to the lower side of the carrier layer; and
(b) applying heat to the lower side of the carrier layer/heat insulating coating.

D1 does not disclose applying heat to the metal layer.

D1 does state that when the mat is used with the heat insulating coating on its lower surface, the mat may be turned upside down after a period of use such that the uncoated surface of the mat comes in contact with the heater. However, in this particular embodiment there is clearly no metal layer.

As such, it can be seen that a need exists for an improved method for dispensing volatile vapors.

### BRIEF SUMMARY OF THE INVENTION

The invention provides a method for dispensing volatile vapors comprising the steps of
(a) providing a mat comprising a carrier layer having a solid carrier material with a volatile material placed therein, the carrier material being constructed and arranged so as to emit the volatile material when heated, and a first metal layer secured to a side of the carrier layer; and
(b) applying heat to the side of the metal layer opposite the carrier layer in an amount sufficient to volatilize the volatile material.

The term "solid" carrier material is meant to distinguish liquids, soft gels, and other materials that require containers for their use on a heater. In contrast, "solid" carrier material is defined to include any material having physical properties such that it does not need to be held in a container, including, by way of example only, felted or woven fibrous materials, solid or foamed polymers, ceramics, and the like. The preferred carrier material is a felted, cellulosic material.

Preferably the first metal layer is secured adhesively, although crimping, riveting, and other ways to attach two layers can be used and would fall within the spirit and scope of the invention. Unless a contrary result is specified, all layers secured to each other, as described herein, can be secured by any of these or equivalent ways of doing so, although adhesive attachment is generally preferred. When a side of the metal layer opposite the carrier material is heated, the metal layer distributes heat along itself and transmits heat to the carrier.

The volatile material is preferably an insecticide, insect repellent, developmental controller, or other insect control material. Alternatively, the volatile material may be a fragrance, deodorizer, or other air quality modifying material. The volatile material can be added to the carrier layer by any convenient method, including but not limited to the conventional methods well known to the art by which active materials are applied to conventional mosquito mats.

The mat may also have a non-metallic layer secured to a side of the first metal layer opposite the carrier layer. The non-metallic layer provides a temperature step down from the temperature of the heater to that experienced by the volatile material. Preferably, there is also a second metal layer secured to a side of the non-metallic layer opposite the first metal layer. This embodiment provides further temperature step down and distribution.

The last layer most distant from the volatile material is preferably a non-metallic layer, which then contacts the heater. This embodiment is particularly desirable for heaters that have poor temperature control.

The mat may have a non-metallic layer with leg portions extending therefrom, preferably extending downwardly, away from the volatile material, and the mat also has a second metal layer adhesively secured to the legs opposite the first metal layer. By this arrangement, a cavity is provided between a portion of the non-metallic layer and the second metal layer. This is particularly desirable with respect to heaters that occasionally provide temperature spikes.

The method of the present invention spreads heat more uniformly across the surface of the mat, as well as reduces excess temperatures that may be developed from poorly controlled heaters or heaters designed for use with less temperature sensitive volatiles.

These and still other features and advantages of the present invention will be apparent from the description which follows. The following description is of the preferred embodiments. The claims should be looked to in order to understand the full scope of the invention.

### BRIEF DESCRIPTION OF THE DRAWINGS

Fig. 1 is a vertical sectional view showing a mat for dispensing volatile materials of the present invention;
Figs. 2, 3, and 4 are views similar to Fig. 1, albeit showing three additional embodiments; and
Fig. 5 is both a left side and frontal view of the Fig. 4 embodiment.

### DETAILED DESCRIPTION

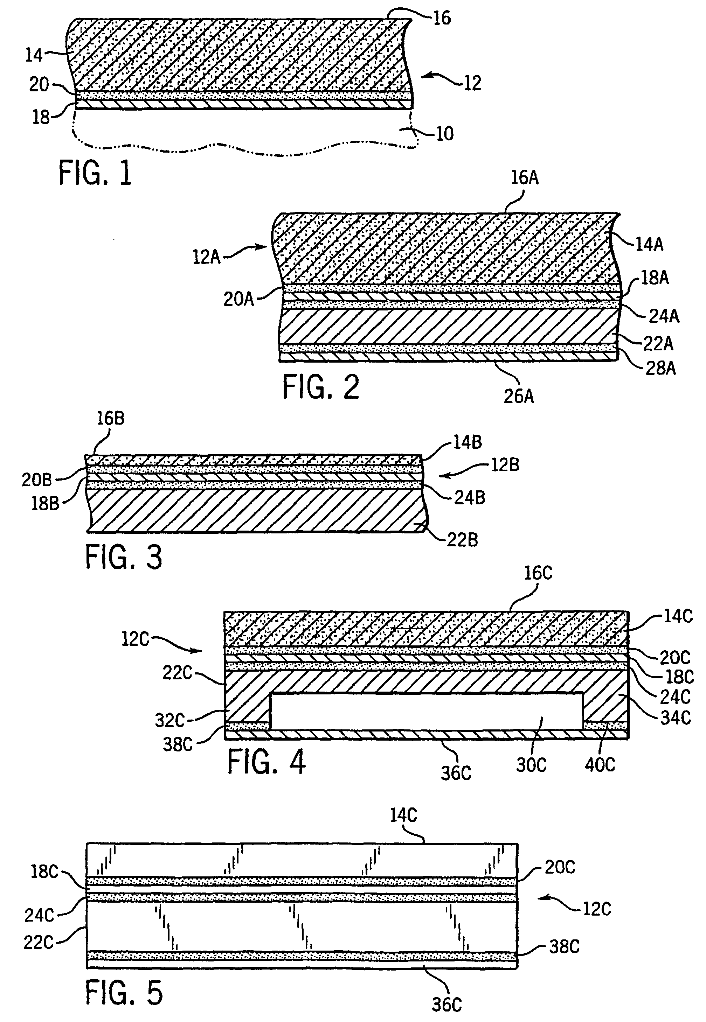

Fig. 1 shows in dotted lines a portion 10 of an electrical heater. The heater can be the FUYI VAPE heater previously described, except that the single layer mat conventionally used with that heater has now been replaced with a mat of the present invention. The FUYI VAPE heater is an electrical-resistance heater having a flat, upwardly exposed plate 10 on/against which is placed a mat generally 12 (or 12A or 12B or 12C) of the present invention. Although the FUYI VAPE heater is shown by way of example, other heaters of any type intended for use with comparable mats could be used instead.

For purposes of convenient description, the hot heater surface will be designated as being in a "down" direction, with features of mats being referred to as being "upper" or "lower" or "above" or "below" each other. In fact, the hot heater surface of some conventional heaters are vertical, but such variation is not important to an understanding of the method of the invention.

Mat 12 has an upper, solid carrier layer 14, preferably made of paper or other porous, cellulose-based material. Other solid porous substrates could also be used, such as sintered glass, plastic beads, ceramic materials, natural or synthetic fabrics, and other absorbent and adsorbent materials. Alternatively, the solid carrier layer 14 can be of a non-porous material so long as it is capable of holding the volatile to be released. Carrier layer 14 is impregnated with or otherwise bears a volatile 16. When placed over the heater the volatile is released from it when mat 12 is heated.

Under the carrier layer 14 is a first layer 18 of metallic material such as aluminum. It is secured to the carrier layer 14, preferably by an adhesive 20. The preferred adhesive is a polymeric adhesive sold as adhesive "711" (RTM) available from Manufacturer Resources Inc. of New Berlin, Wis. Other adhesives could also be used, such as high temperature resistant acrylics and urethanes. When selecting adhesives it is desirable that the adhesive be heat-stable and be able to bond a metal layer to a non-metallic layer. Further, it is preferred that the insulating characteristics of the adhesive be minimal.

The first metal layer 18 distributes heat across itself and then to carrier layer 14. This helps reduce hot spots and to some extent provides a temperature step down.

Figs. 2-5 illustrate alternative embodiments, wherein similar components are indicated with similar reference numbers, except with an A, B, or C suffix.

Referring to Fig. 2, mat 12A includes a carrier layer 14A impregnated with volatile material 16A as well as first and second metal layers 18A and 26A. It also includes a layer 22A of low heat-conductive material such as fiber mat, ceramic, cellulose, etc. If desired and correctly chosen, the same material used for carrier layer 14A also can be used for layer 22A, albeit without the volatile. Preferably, adhesives 20A, 24A and 28A, which can all be the "711" adhesive (RTM) or similar adhesives, secure the layers together. Embodiment 12A offers additional uniform heat distribution as well as an even more pronounced temperature step down.

Embodiment 12B (shown in Fig. 3) is similar to embodiment 12A, except that it does not have the metallic layer 26A or intervening adhesive 28A. Non-metallic material 22B is placed in direct contact with the heater.

Embodiment 12C (shown in Figs. 4 and 5) is somewhat similar to embodiment 12B, except that the low heat-conductive, non-metallic layer 22C has a cavity portion 30C provided by legs 32C and 34C. This cavity provides an air gap permitting even further reduction of hot spots near the center of the mat. Second metal layer 36C extends from one leg 32C to the other 34C, the metal layer being secured to the legs 32C and 34C by adhesive 38C and 40C. The cavity can be open to the air at the sides, or, as shown in the drawings, can be completely enclosed at the sides.

The volatile material is preferably one of (or mixtures of ) known insecticides and insect repellents. Particularly preferred are organic phosphorous insecticides, lipidamide insecticides, natural repellents as citronella oil, natural pyrethrins and pyrethrum extract, and synthetic pyrethroids. Suitable synthetic pyrethroids are allethrin as Pynamin, d-allethrin as Pynamin forte®, benfluthrin, bifenthrin, bioallethrin, S-bioallethrin, esbiothrin, esbiol, bioresmethrin, cycloprothrin, cyfluthrin, beta-cyfluthrin, cyhalothrin, lambda-cyhalothrin, cypermethrin, alpha-cypermethrin, beta-cypermethrin, cyphenothrin, deltamethrin, empenthrin, esfenvalerate, fenpropathrin, fenvalerate, flucythrinate, tau-fluvalinate, kadethrin, permethrin, phenothrin, prallethrin as Etoc®, resmethrin, tefluthrin, tetramethrin, transfluthrin, or tralomethrin. Other volatile insecticides as described in U.S. patent 4,439,415 can also be employed.

Most preferred is Pynamin Forte®. Particularly desirable performance has been achieved by dissolving 40 mg of this active ingredient in 60 mg of the hydrocarbon solvent isopar M (RTM) (from Exxon). 100 mg of the resulting solution is used to impregnate the cellulose portion of a substantially flat mat having 22 mm x 35 mm top view dimension. Preferably the cellulose layer is approximately 2.5 mm thick and is adhered to a .5 mm thick aluminum foil using 10 mg of the 711 (RTM) adhesive prior to impregnation.

Conventionally, piperonyl butoxide is co-delivered with insecticides or other insect control materials as a synergist that improves the insect control effect of the insecticides or other insect control materials. Surprisingly, it has been found that mats having a volatile material that includes an insecticide or other insect control material but that is essentially free of piperonyl butoxide perform as well as conventional, fibrous mats that include both the insecticide or other insect control material and piperonyl butoxide. A mat is defined as being "essentially free of piperonyl butoxide" if it contains piperonyl butoxide at a level less than that level at which an increase in insect control effect can be detected against *Aedes aegypti* mosquitoes when piperonyl butoxide is added to a conventional, fibrous mat bearing the same amount of insecticide or other insect control material as the mat used in the method of the invention. In any event, while piperonyl butoxide amounts of about a 1:1 ratio with insecticide by weight are conventional in the art, a preferred mat is one whose volatile material has less than a ratio of 1:4 by weight of piperonyl butoxide to the weight of insecticide or other insect control material. Most preferably, no piperonyl butoxide is present at all.

Deodorizers may also be used with the mat of the invention, such as a terpene based deodorizer fragrance. Further, volatile fragrances, disinfectants, or other air quality modifying agents may be used, such as glycols, trimethylene, and dipropylene. In addition, organic acids that are compatible with the use of the substrate and the atmosphere can also be utilized.

The method of the invention for controlling insects includes a first steps of providing a mat having a solid carrier layer having placed therein a volatile material including an insect control material, the carrier layer being constructed and arranged so as to emit the volatile material when heated; the mat also having a first metal layer secured to a side of the carrier layer. The second step of the method is to apply heat to the first metal layer in an amount sufficient to volatilize the insect control material.

The heat and amount of volatile material must be sufficient to volatilize insect control material in amounts sufficient to achieve the desired amount of insect control. The specific amount of volatile material and heat will depend on the insect control material used and volume of space within which insects are to be controlled. Determining such parameters is well within the skill of the person skilled in this art. Preferably, the step of providing a mat includes providing a mat wherein the volatile material is essentially free of piperonyl butoxide, as the term "essentially free of piperonyl butoxide" has been defined, above. Alternatively, that step includes providing a mat wherein the volatile material includes no more than a ratio of 1:4 by weight of piperonyl butoxide to the insecticide or other insect control material. Preferably, that step includes providing a mat entirely free of piperonyl butoxide.

The invention is not to be limited to the specific embodiments shown. Rather, the claims should be looked to in order to appreciate the full scope of the claimed invention.

### INDUSTRIAL APPLICABILITY

The invention provides a method for dispensing volatile materials such as insecticides. The method is particularly useful in controlling mosquitoes over extended periods. The invention also includes practical methods for controlling mosquitoes.

## Claims

1. A method for dispensing volatile vapors comprising the steps of
(a) providing a mat comprising a carrier layer having a solid carrier material with a volatile material placed therein, the carrier material being constructed and arranged so as to emit the volatile material when heated, and a first metal layer secured to a side of the carrier layer; and
(b) applying heat to the side of the metal layer opposite the carrier layer in an amount sufficient to volatilize the volatile material.

2. The method of claim 1 wherein the volatile material is selected from insecticides, insect repellents, insect developmental controllers and combinations thereof.

3. The method of claim 1 or 2 wherein the solid carrier material is a cellulosic material.

4. The method of any preceding claim wherein the mat additionally comprises a layer of low heat-conductive material secured to the side of the first metal layer opposite the carrier layer.

5. The method of claim 4 wherein the mat additionally comprises a second metal layer secured to the side of the layer of low heat-conductive material opposite the first metal layer.

6. The method of claim 5 wherein the layer of low heat-conductive material has a cavity portion (30c) provided by legs (32c and 34c).

7. The method of any preceding claim wherein the volatile material includes an insect control material that is essentially free of piperonyl butoxide.

8. The method of any one of claims 1-6 wherein the volatile material includes an insect control material that includes no more than a 1:4 ratio by weight of piperonyl butoxide to insect control material.

9. The method of any one of claims 1-6 wherein the volatile material includes an insect control material that is entirely free of piperonyl butoxide.

## Patentansprüche

1. Verfahren zur Abgabe flüchtiger Dämpfe mit folgenden Schritten:
a. Bereitstellen einer Matte mit einer Trägerschicht mit einem festen Trägermaterial, in das ein flüchtiger Stoff eingebracht ist und das so aufgebaut und angeordnet ist, dass der flüchtige Stoff bei Erwärmung freigesetzt wird, sowie mit einer ersten Metallschicht, die auf einer Seite der Trägerschicht befestigt ist; und
b. Aufbringen von Wärme in einer zum Verdampfen des flüchtigen Stoffs ausreichenden Menge auf die der Trägerschicht entgegengesetzte Seite der Metallschicht.

2. Verfahren nach Anspruch 1, bei dem der flüchtige Stoff gewählt ist aus den Insektiziden, den Insekten abweisenden und den die Entwicklung von Insekten beeinflussenden Substanzen sowie deren Kombinationen.

3. Verfahren nach Anspruch 1 oder 2, bei dem das feste Trägermaterial ein Cellulose-Material ist.

4. Verfahren nach einem der vorgehenden Ansprüche, bei dem die Matte zusätzlich eine Schicht aus einem Material niedriger Wärmeleitfähigkeit aufweist, das an der der Trägerschicht entgegengesetzten Seite der ersten Metallschicht befestigt ist.

5. Verfahren nach Anspruch 4, bei dem die Matte zusätzlich eine zweite Metallschicht aufweist, die an der der ersten Metallschicht entgegengesetzten Seite der Schicht aus einem Material niedriger Wärmeleitfähigkeit befestigt ist.

6. Verfahren nach Anspruch 5, bei dem die Schicht aus einem Material niedriger Wärmeleitfähigkeit einen Hohlraumbereich (30c) aufweist, der von Beinen (32c und 34c) erzeugt wird.

7. Verfahren nach einem der vorgehenden Ansprüche, bei dem der flüchtige Stoff mindestens ein Insektenbekämpfungsmaterial aufweist, das im Wesentlichen frei von Piperonylbutoxid ist.

8. Verfahren nach einem der Ansprüche 1 - 6, bei dem der flüchtige Stoff ein Insektenbekämpfungsmaterial aufweist derart, dass das Gewichtsverhältnis des Piperonylbutoxids zum Insektenbekämpfungsmaterial nicht mehr als 1:4 beträgt.

9. Verfahren nach einem der Ansprüche 1 - 6, bei dem der flüchtige Stoff ein Insektenbekämpfungsmaterial aufweist, das vollständig frei von Piperonylbutoxid ist.

## Revendications

1. Procédé de distribution de vapeurs volatiles comprenant les étapes suivantes :
(a) la disposition d'un feutre ayant une couche de support contenant un matériau solide de support avec une matière volatile placée à l'intérieur, le matériau de support ayant une construction et une disposition telles qu'il émet la matière volatile lorsqu'il est chauffé, et une première couche métallique fixée à un côté de la couche de support, et
(b) l'application de chaleur au côté de la couche métallique opposée à la couche de support en quantité qui suffit pour la volatilisation de la matière volatile.

2. Procédé selon la revendication 1, dans lequel la matière volatile est sélectionnée parmi les insecticides, les agents répulseurs d'insectes, les agents de contrôle du développement des insectes et leurs combinaisons.

3. Procédé selon la revendication 1 ou 2, dans lequel le matériau solide de support est un matériau cellulosique.

4. Procédé selon l'une quelconque des revendications précédentes, dans lequel le feutre comporte en outre une couche d'un matériau peu conducteur de la chaleur, fixé au côté de la première couche métallique opposé à la couche de support.

5. Procédé selon la revendication 4, dans lequel le feutre comporte en outre une seconde couche métallique fixée au côté de la couche du matériau peu conducteur de la chaleur opposé à la première couche métallique.

6. Procédé selon la revendication 5, dans lequel la couche du matériau peu conducteur de la chaleur a une partie de cavité (30c) formée par des pieds (32c et 34c).

7. Procédé selon l'une quelconque des revendications précédentes, dans lequel la matière volatile comprend une matière de lutte contre les insectes qui est essentiellement dépourvue de butoxyde de pipéronyle.

8. Procédé selon l'une quelconque des revendications 1 à 6, dans lequel la matière volatile contient une matière de lutte contre les insectes qui contient une quantité pondérale de butoxyde de pipéronyle qui ne dépasse pas un rapport 1/4 par rapport à la matière de lutte contre les insectes.

9. Procédé selon l'une des revendications 1 à 6, dans lequel la matière volatile contient une matière de lutte contre les insectes qui est entièrement dépourvue de butoxyde de pipéronyle.
